Europäisches Patentamt

European Patent Office

Office européen des brevets

(11) Veröffentlichungsnummer : **0 058 976**
**B1**

(12) # EUROPÄISCHE PATENTSCHRIFT

(45) Veröffentlichungstag der Patentschrift :
03.04.85

(21) Anmeldenummer : 82101317.4

(22) Anmeldetag : 20.02.82

(51) Int. Cl.⁴ : **C 07 C 19/045, C 07 C 17/156**

(54) **Verfahren zur Herstellung von 1,2-Dichlorethan.**

(30) Priorität : 25.02.81 DE 3106983

(43) Veröffentlichungstag der Anmeldung :
01.09.82 Patentblatt 82/35

(45) Bekanntmachung des Hinweises auf die Patenterteilung : 03.04.85 Patentblatt 85/14

(84) Benannte Vertragsstaaten :
**BE DE FR GB IT NL SE**

(56) Entgegenhaltungen :
EP-A- 0 019 863
DE-A- 2 364 095
US-A- 4 172 099
**Die Akte enthält technische Angaben, die nach dem
Eingang der Anmeldung eingereicht wurden und die
nicht in dieser Patentschrift enthalten sind.**

(73) Patentinhaber : **HOECHST AKTIENGESELLSCHAFT
Postfach 80 03 20
D-6230 Frankfurt am Main 80 (DE)**

(72) Erfinder : **Kühn, Wenzel, Dr.
Kampenwandstrasse 15
D-8261 Burgkirchen/Alz (DE)**
Erfinder : **Widmann, Peter
Anna-Schäffer-Strasse 18
D-8261 Emmerting (DE)**

**Beschreibung**

Die Erfindung betrifft ein Verfahren zur Herstellung von 1,2-Dichlorethan, wobei Ethylen mit Chlorwasserstoff, Sauerstoff enthaltenden Inertgasen und Chlor in einem gemeinsamen Reaktionsraum in Gegenwart eines Katalysators umgesetzt wird.

1,2-Dichlorethan wird bereits seit einer Reihe von Jahren in großem Maße industriell hergestellt. Es wird hauptsächlich durch thermische Spaltung zu Vinylchlorid umgesetzt, welches wiederum die Grundlage für den Massenkunststoff Polyvinylchlorid ist. Diese Verwendung hat 1,2-Dichlorethan zu einem der am meisten produzierten aliphatischen, chlorierten Kohlenwasserstoffe gemacht. Zu seiner Herstellung sind eine Reihe verschiedener Verfahren bekannt, von denen die meisten von Ethylen ausgehen. Im allgemeinen wird an Ethylen direkt elementares Chlor angelagert, wobei man bei Temperaturen von 40 bis etwa 120 °C in flüssiger Phase häufig in 1,2-Dichlorethan arbeitet. In einer viel gebrauchten Form dieses Verfahrens wird die bei der Chloranlagerung entstehende beträchtliche Wärmemenge durch siedendes 1,2-Dichlorethan abgeführt. Da der Siedepunkt des 1,2-Dichlorethans bei normalem Atmosphärendruck bei etwa 84 °C liegt, reicht das Temperaturniveau, bei dem die Wärmemenge abgeführt wird, entweder nicht aus, um Wasserdampf zu erzeugen oder es kann Wasserdampf nur bei niedriger Temperatur und damit niedrigem Druckniveau erhalten werden, der zur Wiederverwendung der in ihm enthaltenen Energie nur beschränkt einsatzfähig ist.

Zur besseren Ausnutzung der Reaktionswärme der direkten Chloranlagerung an Ethylen ist es bekannt, die Umsetzung in der Gasphase in Gegenwart eines aufgewirbelten Katalysators durchzuführen und unmittelbar anschließend das gebildete 1,2-Dichlorethan zu Vinylchlorid zu spalten. Die Katalysatorteilchen wirken hierbei als Wärmeüberträger, es wird bei Temperaturen von 370 bis 540 °C und Drücken bis 2,2 MPa, vorzugsweise bei 0,45 bis 1,85 MPa, gearbeitet. Der bei der Spaltung von 1,2-Dichlorethan entstehende Chlorwasserstoff wird in einem getrennten Apparat für die Oxychlorierung von Ethylen verwendet. Das hieraus gewonnene 1,2-Dichlorethan wird in den Wirbelbett-Spaltreaktor zurückgeleitet.

Nachteile des Verfahrens, wie die Bildung beträchtlicher Mengen Ethylenchlorid, verhältnismäßig große Anteile an nichtumgesetztem 1,2-Dichlorethan in den Spaltprodukten, Schwierigkeiten bei der Regelung und Steuerung des Prozesses, Tendenz zur Bildung unerwünschter polychlorierter Kohlenwasserstoffe und zur Verharzung und Verkokung des Spaltreaktors, sollen vermindert werden, wenn anstelle von beispielsweise Dehydrochlorierungskatalysatoren fluidisierte, nichtkatalytische Feststoffe in dem Reaktor verwendet werden. Ferner muß das Chlor in der Chlorierungsreaktionszone an einer Anzahl verschiedener Stellen kontrolliert eingeführt werden, um die Verkokungsgefahr herabzusetzen. Hierzu ist ein besonderer, relativ aufwendiger Einbau in den Wirbelbett-Spaltreaktor erforderlich. Es besteht ferner die Schwierigkeit, die heißen Spaltgase vom aufgewirbelten, feinteiligen, festen Wärmeübertragungsmedium möglichst vollständig zu trennen, und der Nachteil, daß bei diesen Verfahren nicht der industriell bevorzugte Röhrenspaltofen für flüssiges oder gasförmiges 1,2-Dichlorethan verwendet werden kann, der hohe Durchsatzleistungen ermöglicht.

Es ist ferner ein Verfahren zur Herstellung von 1,2-Dichlorethan bekannt, wobei in einer ersten Stufe überschüssiges Ethylen, Chlorwasserstoff und überschüssiger Sauerstoff in Form von Luft in Gegenwart eines bekannten Oxychlorierungskatalysators durch entsprechende Einstellung der Mengenverhältnisse der Ausgangsstoffe unter mehr als 90 %igem Umsatz an Chlorwasserstoff bei 180 bis 350 °C zur Reaktion gebracht werden und die Restgase aus dieser Stufe nach Auswaschen des nichtumgesetzten Chlorwasserstoffs und der dadurch bedingten Kondensation eines Großteils des entstandenen 1,2-Dichlorethans in einer zweiten Stufe mit 80 bis 120 Mol-% Chlor, bezogen auf das in dieser Stufe eingesetzte Ethylen, in Gegenwart eines eisenhaltigen Katalysators bei 80 bis 250 °C umgesetzt werden.

Ein ähnliches Verfahren arbeitet ebenfalls in zwei getrennten Stufen, zwischen denen 1,2-Dichlorethan und Wasser aus dem Reaktionsprodukt abgeschieden werden, wobei in der zweiten Stufe das überschüssige Ethylen aus der Oxychlorierung mit Chlor bei einer Temperatur von 80 bis 320 °C in Gegenwart eines aktivierten Aluminiumoxid-Katalysators umgesetzt wird.

Neuerdings wurde bekannt, die bei der Nachchlorierung des überschüssigen Ethylens aus der Oxychlorierung gebildeten erheblichen Mengen an 2-Chlorethanol dadurch herabzusetzen, daß man die Umsetzung in Gegenwart von zugesetztem Chlorwasserstoff durchführt.

Schließlich ist ein Verfahren bekannt, die Nebenprodukte bei der Chlorierung von ethylenhaltigen Gasen, die nach der Oxychlorierung und Abtrennung der Hauptmenge der gebildeten organischen Produkte durch Abschrecken mit Wasser anfallen, dadurch zu vermindern, daß man die Chlorierung in Gegenwart von Kupfer (II)-chlorid und/oder Eisen (III)-chlorid auf einem Träger als Katalysator durchführt.

Alle diese letztgenannten Verfahren haben den Nachteil, daß ein zusätzlicher Reaktor mit Produktabscheider und weiteren Einrichtungen benötigt wird, um die Ausbeute an 1,2-Dichlorethan, bezogen auf das eingesetzte Ethylen, bei der Oxychlorierung zu verbessern.

Neuerdings wurde aus der EP-A-0 019 863 ein Verfahren bekannt, mit dem man sowohl die Oxychlorierung wie auch die direkte Chloranlagerung an Ethylen in einem gemeinsamen Reaktionsraum mit guten Ausbeuten an 1,2-Dichlorethan ausführen kann, wobei das Temperaturniveau der Reaktion eine wesentlich bessere Ausnutzung der abgeführten Reaktionswärme der Chloranlagerung an Ethylen ermöglicht, als nach dem bisher viel verwendeten Verfahren der Chlorierung in siedendem 1,2-Dichlorethan.

Gegenstand der vorliegenden Erfindung ist eine Weiterentwicklung des zuletzt genannten Verfahrens, die eine gleichmäßigere Temperaturführung im gemeinsamen Reaktionsraum ermöglicht.

Das neue Verfahren zur Herstellung von 1,2-Dichlorethan aus Ethylen durch Umsetzung mit Chlorwasserstoff und Sauerstoff enthaltenden Inertgasen bei 180 bis 300 °C und 0,1 bis 1,1 MPa sowie durch Umsetzung mit Chlor in der Gasphase in Gegenwart eines kupfer- oder kupfer- und eisensalzhaltigen, festen Katalysators mit anschließender Abkühlung und destillativer Auftrennung des Reaktionsgemisches, wobei beide Chlorierungsreaktionen in einem gemeinsamen Reaktionsraum, der aufgewirbelte Katalysatorteilchen enthält, nacheinander durchgeführt werden, und die im gesamten Reaktionsraum gebildete Wärme durch indirekte Kühlung mit einem flüssigen und/oder gasförmigen Wärmeübertragungsmedium abgeführt wird, ist dadurch gekennzeichnet, daß 98 bis 40 % der Gesamtmenge des in den gemeinsamen Reaktionsraum eingeleiteten Ethylenvolumens in die Zone des Reaktionsraumes eingeleitet wird, in der die erste Chlorierungsreaktion stattfindet, und die restlichen 2 bis 60 % der Gesamtmenge des in den gemeinsamen Reaktionsraum eingeleiteten Ethylen-Volumens in die Zone des Reaktionsraumes eingeleitet wird, in der die zweite Chlorierungs-Reaktion stattfindet.

Die beiden Chlorierungs-Reaktionen können in beliebiger Reihenfolge nacheinander durchgeführt werden, wobei ein qualitativ hochwertiges 1,2-Dichlorethan in guten Ausbeuten auch dann erhalten wird, wenn die umgesetzte Menge Ethylen bei jeder Chlorierungsreaktion etwa gleich groß ist. Letzteres Verfahren ist eine bevorzugte Variante des erfindungsgemäßen Verfahrens, wenn das erzeugte 1,2-Dichlorethan anschließend wie üblich durch thermische Spaltung zu Vinylchlorid umgesetzt wird, da auf diese Weise die Gesamtmenge des zu spaltenden 1,2-Dichlorethans in einer Reaktionseinheit unter guter Wärmeausnutzung erzeugt werden kann, wobei der durch den Spaltprozeß erzeugte Chlorwasserstoff in die 1,2-Dichlorethan-Herstellung zurückgeführt und zur Chlorierung von etwa der Hälfte des insgesamt eingesetzten Ethylens verwendet wird.

Als Wärmeübertragungsmedium für die Abführung der im gesamten Reaktionsraum gebildeten Wärme durch indirekte Kühlung eignen sich beispielsweise hochsiedende Mineralöle und Silikonöle. Bevorzugt wird Wasser eingesetzt, das durch Wärmeaufnahme in Mitteldruckdampf verwandelt wird.

In einer bevorzugten Ausführungsform des erfindungsgemäßen Verfahrens wird in den Reaktionsraum zuerst das Sauerstoff enthaltende Inertgas, eine erste Teilmenge des Ethylens und der Chlorwasserstoff sowie danach die zweite Teilmenge des Ethylens und Chlor in folgenden Molverhältnissen eingeführt :

2 mol HCl ; 1,01 bis 3 mol $C_2H_4$ (Gesamtmenge) ; mindestens 0,5, im allgemeinen 0,5 bis 1 mol $O_2$ und 0,009 bis 2 mol $Cl_2$, wobei die Chlormenge so bemessen wird, daß im Endprodukt der Reaktion, das heißt im Gasgemisch, das den Reaktionsraum verläßt, weniger als 0,001 Gew.-% freies, elementares Chlor gefunden werden.

Die im vorhergehenden Absatz angegebene Gesamtmenge des Ethylens von 1,01 bis 3 mol wird so aufgeteilt, daß 98 bis 40 % dieser Gesamtmenge, vorzugsweise 95 bis 60 % dieser Gesamtmenge, zusammen mit dem Chlorwasserstoff oder in dessen unmittelbarer Nähe in den Reaktionsraum eingeleitet werden. Die restlichen 2 bis 60 %, vorzugsweise 5 bis 40 % der Gesamtmenge des Ethylens werden in der Nähe, zweckmäßig kurz vor der Stelle, an der Chlor in den Reaktionsraum eingeführt wird, in den Reaktionsraum eingeleitet. Wenn beispielsweise die Gesamtmenge des in einer bestimmten Zeit in den Reaktionsraum eingegebenen Ethylens 2 mol beträgt, so werden davon 98 bis 40 %, das sind 1,96 bis 0,8 mol, vorzugsweise 95 bis 60 %, das sind 1,9 bis 1,2 mol, Ethylen zusammen mit dem Chlorwasserstoff eingeleitet und 2 bis 60 %, das sind 0,04 bis 1,2 mol, vorzugsweise 5 bis 40 %, das sind 0,1 bis 0,8 mol, Ethylen kurz vor der Chlor-Einführung in den Reaktionsraum eingeleitet. Wenn weniger als 40 % der Gesamtmenge des Ethylens zusammen mit dem Chlorwasserstoff oder in dessen unmittelbarer Nähe eingeleitet werden, wird eine Abnahme der Ausbeute an 1,2-Dichlorethan und Zunahme der Bildung von unerwünschten Nebenprodukten beobachtet.

Unter Inertgas sind solche Stoffe zu verstehen, die unter den Reaktionsbedingungen gasförmig sind und an der Reaktion entweder überhaupt nicht oder in ganz untergeordnetem Maße teilnehmen. Beispiele für Inertgase sind Stickstoff, Kohlendioxid und 1,2-Dichlorethan-Dampf. Bevorzugt wird Stickstoff als Inertgas eingesetzt. Die Inertgas-Menge wird zweckmäßig so bemessen, daß eine ausreichende Aufwirbelung der festen Katalysatorteilchen erreicht wird, ohne das Reaktionsgemisch zu weitgehend zu verdünnen.

Bevorzugt wird der Sauerstoff zu mindestens 50 bis 100 %, insbesondere zu 90 bis 100 %, seiner Gesamtmenge als Luft in den Reaktionsraum eingeleitet.

Alle Gase werden möglichst mit geringer relativer Feuchte in die Reaktionszone gegeben. Das Chlorwasserstoffgas stammt bevorzugt aus der thermischen Spaltung von 1,2-Dichlorethan zur Herstellung von Vinylchlorid. Vor Eingabe in den Reaktionsraum können die Gase beispielsweise auf Temperaturen von 60 bis 180 °C vorgeheizt werden.

Alle Gase können einzeln, das Ethylen in mindestens zwei Teilmengen in den Reaktionsraum eingeführt werden, bevorzugt werden jedoch Chlorwasserstoff und eine erste Teilmenge des Ethylens einerseits sowie Sauerstoff und Inertgas, beispielsweise als Luft, andererseits, jeweils in Mischung miteinander, eingeführt. Das Chlor kann bei diskontinuierlicher Fahrweise zeitlich, bei der bevorzugten kontinuierlichen Fahrweise räumlich nach der Eingabe der anderen Gase in den Reaktionsraum eingeführt werden. Zweckmäßig kurz vor der Chloreinführung wird die zweite Teilmenge des Ethylens in den Reaktionsraum eingespeist.

Der Reaktionsraum kann beispielsweise kugelige, ellipsoidische oder zylindrische Gestalt haben, er sollte so ausgebildet sein, daß er keine toten Ecken und Winkel aufweist, in denen sich der aufgewirbelte Katalysator absetzen kann. Vorzugsweise wird ein langgestreckt zylindrischer Reaktionsraum mit kreisförmigem Querschnitt und senkrecht stehender Zylinderachse, beispielsweise ein Rohr, verwendet.

Der Reaktionsraum ist zweckmäßig mit einem Doppelmantel sowie Einbauten ausgerüstet, durch die das Wärmeübertragungsmedium fließt. Geeignete Einbauten sind beispielsweise Schlangen- oder Rohrregister-Kühler. Diese Einbauten können in mehreren, voneinander getrennten Einheiten angeordnet sein, die von verschiedenen Medien mit unterschiedlichen Strömungsgeschwindigkeiten durchflossen werden können, um eine optimale Wärmeausnutzung und einen optimalen Temperaturverlauf im Reaktionsraum zu ermöglichen.

Die verschiedenen Gase können durch einfache Rohre in den Reaktionsraum geleitet werden, die zweckmäßig an ihren Enden Einrichtungen zur besseren Verteilung über die Fläche enthalten. Solche geeigneten Einrichtungen sind beispielsweise gelochte Platten oder Kugeln, Fritten oder ein beziehungsweise mehrere Rohre mit einer Vielzahl von Gasausström-Öffnungen.

Der Reaktionsraum enthält zweckmäßig in seiner obersten Zone eine Öffnung mit regelbarem Querschnitt, durch die die Reaktionsprodukte abgeführt werden. Nach Verlassen des Reaktionsraumes passieren die Reaktionsprodukte zweckmäßig einen Abscheider für feinteilige, feste Katalysatorteilchen, beispielsweise einen Zyklon oder ähnlichen Apparat. Die abgeschiedenen Teilchen werden in den Reaktor zurückgeführt.

Nach Verlassen des Abscheiders werden die Gase gegebenenfalls gewaschen und teilweise kondensiert, die bei Normaldruck bei ca. 10 °C nicht kondensierbaren Gasanteile werden, gegebenenfalls nach Abtrennung schädlicher oder störender Stoffe, in die Atmosphäre geleitet. Zumindest ein Teil der nicht-kondensierbaren Gase kann auch im Kreis in den Reaktionsraum zurückgeführt werden. Die kondensierten Reaktionsprodukte werden zwecks Gewinnung von reinem 1,2-Dichlorethan destillativ aufgetrennt, wie üblich.

In einer besonders bevorzugten Ausführungsform der vorliegenden Erfindung werden die umzusetzenden Stoffe in den Reaktionsraum in folgenden Mengenverhältnissen eingeführt:

2 mol HCl; 1,8 bis 2,2 mol $C_2H_4$ (Gesamtmenge); 0,5 bis 0,6 mol $O_2$ und 0,79 bis 1,2 mol $Cl_2$, wobei die Chlormenge so bemssen wird, daß im Gasgemisch, welches den Reaktionsraum verläßt, weniger als 0,001 Gew.-% freies, elementares Chlor gefunden werden. Bei Anwendung dieser Molverhältnisse kann 1,2-Dichlorethan für die nachfolgende Spaltung zu Vinylchlorid unter optimaler Ausnutzung des aus dem Spaltprozeß zurückgeführten Chlorwasserstoffes sowie der Einsatzstoffe Ethylen und Chlor in einer einzigen Reaktionseinheit hergestellt werden.

Wie vorstehend beschrieben wird die Gesamtmenge des Ethylens von 1,8 bis 2,2 mol so aufgeteilt, daß 98 bis 40 % dieser Gesamtmenge, vorzugsweise 95 bis 60 % dieser Gesamtmenge, zusammen mit dem Chlorwasserstoff oder in dessen unmittelbarer Nähe in den Reaktionsraum eingeleitet werden. Die restlichen 2 bis 60 %, vorzugsweise 5 bis 40 %, der Gesamtmenge des Ethylens werden in der Nähe, zweckmäßig kurz vor der Stelle, an der Chlor in den Reaktionsraum eingeführt wird, in den Reaktionsraum eingespeist.

Das in den beiden vorangegangenen Absätzen beschriebene Verfahren wird insbesondere so ausgeführt, daß an einem Ende eines rohrförmigen Reaktors, zweckmäßig am unteren Ende eines senkrecht oder nahezu senkrecht stehenden rohrförmigen Reaktors eine erste Teilmenge des Ethylens, Chlorwasserstoff und sauerstoffhaltiges Inertgas getrennt oder zumindest teilweise getrennt voneinander eingeleitet werden. Es können beispielsweise die erste Teilmenge des Ethylens und Chlorwasserstoff gemeinsam, jedoch getrennt vom sauerstoffhaltigen Inertgas eingeführt werden. In einem bestimmten Abstand von der in Gasströmungsrichtung letzten der obengenannten Gaseinleitungen entfernt wird Chlor und zweckmäßig kurz davor die zweite Teilmenge des Ethylens in den Reaktionsraum eingeleitet. Die Lage der Chloreinleitung wird so gewählt, daß zwischen ihr und der vorhergehenden Chlorwasserstoffeinleitung ein Reaktionsraum liegt, der 40 bis 85 %, vorzugsweise 55 bis 75 %, des gesamten zur Verfügung stehenden Reaktionsraumes im Reaktor beträgt. Am anderen Ende des Reaktors, zweckmäßig am oberen Ende eines senkrechten oder nahezu senkrecht stehenden rohrförmigen Reaktors, werden die Reaktionsprodukte abgeführt.

Ein solches Verfahren ist insbesondere für den technisch wichtigen kontinuierlichen Betrieb geeignet. Für diesen kontinuierlichen Betrieb mit in Strömungsrichtung der Gase zuerst eingeleitetem Chlorwasserstoff und nachfolgend eingeleitetem Chlor wird vorzugsweise das Wärmeübertragungsmedium in der indirekten Kühlung des Reaktionsraums im Gegenstrom zu den im Reaktionsraum befindlichen Gasen geführt. Hierdurch wird eine bessere Wärmeabführung und ein günstigerer Temperaturverlauf im Reaktionsraum erreicht.

Nach einer weiteren bevorzugten Ausführungsform des erfindungsgemäßen Verfahrens werden in den Reaktionsraum zuerst Inertgas, das gegebenenfalls Sauerstoff enthalten kann, sowie eine erste Teilmenge des Ethylens und getrennt davon Chlor sowie nachfolgend Chlorwasserstoff, die zweite Teilmenge des Ethylens und gegebenenfalls Sauerstoff und Inertgas in folgenden Molverhältnissen eingeführt:

4

2 mol C$_2$H$_4$ (Gesamtmenge) ; 0,9 bis 1,2 mol Cl$_2$ ; 1,6 bis 2,3 mol HCl und 0,35 bis 1,3 mol Gesamt-O$_2$, wobei die Sauerstoff- oder die Chlorwasserstoffmenge so bemessen wird, daß im Endprodukt, das heißt im Gasgemisch, das den Reaktionsraum verläßt, weniger als 0,001 Gew.-% freies, elementares Chlor gefunden werden.

Die insgesamt 2 mol in einer bestimmten Zeit eingesetztes Ethylen werden so aufgeteilt, daß 98 bis 40 % dieser Gesamtmenge in unmittelbarer Nähe der Chloreinführung in den Reaktionsraum eingeleitet werden. Die restlichen 2 bis 60 % der Ethylen-Gesamtmenge werden entweder zusammen mit dem Chlorwasserstoff oder in dessen unmittelbarer Nähe in den Reaktionsraum eingespeist.

Diese Ausführungsform des Verfahrens wird insbesondere angewendet, wenn, beispielsweise zur Verminderung des Anteils an 2-Chlorethanol in den Reaktionsprodukten, mit einem geringen Überschuß an Chlorwasserstoff gefahren werden soll. Wie bereits oben beschrieben, ist auch diese Ausführungsform geeignet, 1,2-Dichlorethan für die thermische Spaltung zu Vinylchlorid zu erzeugen, unter optimaler Ausnutzung des bei dieser Spaltung erzeugten Chlorwasserstoffs, wobei die gesamte 1,2-Dichlorethanerzeugung in einem einzigen Reaktionsraum stattfindet.

Als Inertgas sind wiederum, wie oben beschrieben, beispielsweise Stickstoff, Kohlendioxid und/oder 1,2-Dichlorethan-Dampf geeignet, wobei bevorzugt Stickstoff eingesetzt wird. Die Hauptmenge des benötigten Sauerstoffs wird zweckmäßig an der Stelle zugeführt, an der auch der Chlorwasserstoff eingeleitet wird, jedoch ist auch die Zuführung beträchtlicher Mengen Sauerstoff bereits an der Stelle, an der eine erste Teilmenge des Ethylens und Chlor eingeleitet werden, möglich. Diese Verfahrensweise wird insbesondere dann angewendet, wenn als kostengünstiges Wirbelgas Luft eingesetzt werden soll.

Das in den vier vorangegangenen Absätzen beschriebene Verfahren wird insbesondere so ausgeführt, daß an einem Ende eines rohrförmigen Reaktors, zweckmäßig am unteren Ende eines senkrecht oder nahezu senkrecht stehenden rohrförmigen Reaktors eine erste Teilmenge des Ethylens, Chlor und Inertgas, das gegebenenfalls Sauerstoff enthalten kann, zumindest teilweise getrennt voneinander eingeleitet werden. Das Inertgas kann beispielsweise auch in Mischung mit Chlor, jedoch die erste Teilmenge des Ethylens getrennt hiervon, eingeführt werden. In einem bestimmten Abstand von der in Gasströmungsrichtung letzten der obengenannten Gaseinleitungen entfernt wird Chlorwasserstoff und gegebenenfalls Sauerstoff und Inertgas getrennt oder zumindest teilweise getrennt in den Reaktionsraum eingeleitet. Die Lage der Chlorwasserstoffeinleitung wird so gewählt, daß zwischen ihr und der vorhergehenden Chloreinleitung ein Reaktionsraum liegt, der 10 bis 40 %, vorzugweise 15 bis 30 %, des gesamten zur Verfügung stehenden Reaktionsraumes im Reaktor beträgt. Die zweite Teilmenge des Ethylens wird zusammen mit dem Chlorwasserstoff oder in dessen unmittelbarer Nähe in den Reaktionsraum eingeführt. Am anderen Ende des Reaktors, zweckmäßig am oberen Ende eines senkrechten oder nahezu senkrecht stehenden rohrförmigen Reaktors werden die Reaktionsprodukte abgeführt.

Bei der soeben beschriebenen Ausführungsform des neuen Verfahrens wird das Wärmeübertragungsmedium in der indirekten Kühlung des Reaktionsraums vorteilhaft im Gleichstrom zu den Gasen geführt.

Das erfindungsgemäße Verfahren wird vorteilhaft bei Temperaturen des Reaktionsgemisches im Reaktionsraum von 190 bis 250 °C, insbesondere bei 200 bis 230 °C, durchgeführt. Hierbei kann, insbesondere bei kontinuierlichen Verfahren ein räumlicher Temperaturgradient angewendet werden. Beispielsweise kann an der Einführungsstelle der Gase in den Reaktionsraum eine niedrigere Temperatur herrschen als an der Entnahmestelle der Reaktionsprodukte. Die Temperatur kann auch in Strömungsrichtung der Gase gesehen im ersten Drittel des Reaktionsraumes oder in der Mitte oder im zweiten Drittel höher sein als in den restlichen Bereichen des Reaktionsraumes.

Zweckmäßig werden die Gase vor Einführung in den Reaktionsraum auf Temperaturen von 50 bis etwa 180 °C erwärmt.

Das neue Verfahren kann bei normalem Atmosphärendruck (0,09 bis 0,1 MPa) durchgeführt werden. Im allgemeinen wird man zur Erhöhung der Raum-Zeit-Ausbeute erhöhten Druck bis etwa 1,1 MPa anwenden. Vorzugsweise wird bei Drücken von 0,3 bis 0,6 MPa gearbeitet.

Der feste Katalysator wird vorteilhaft in feinteiliger Form mit einer mittleren Teilchengröße von 20 bis 400 μm angewendet. Besonders gute Ergebnisse werden mit einem Katalysator mit einer mittleren Teilchengröße von 30 bis 70 μm erhalten.

Der Katalysator besteht zweckmäßig aus einer Trägersubstanz, die eine große Oberfläche je Gewichtseinheit, beispielsweise 70 bis 200 m$^2$/g und mehr, aufweist, bei hohen Temperaturen, beispielsweise bis mindestens 500 °C, mechanisch stabil ist und aus der Gasreaktion unverändert hervorgeht. Geeignete Trägermaterialien sind wärmebeständige Oxide, beispielsweise Siliciumdioxid oder Aluminiumoxid sowie Diatomeenerde oder silikatische Materialien. Vorzugsweise wird Aluminiumoxid eingesetzt.

Auf diesem Trägermaterial ist zweckmäßig etwa 0,5 bis 15 Gew.-%, bezogen auf den gesamten Katalysator, Kupfer als Salz oder Oxid aufgebracht. Diese Kupfersalze bzw. -oxide verwandeln sich in der Regel während des Gebrauchs durch den anwesenden Chlorwasserstoff und das Chlor in Kupfer (II)-chlorid, sofern sie nicht schon von Anfang an als dieses Chlorid aufgebracht wurden.

Neben Kupfer kann der Katalysator mit Vorteil noch kleinere Mengen von Lewis-Säuren, insbesondere etwa 0,01 bis 0,5 Gew.-%, bezogen auf den gesamten Katalysator, Eisen, das als Oxid oder als Salz aufgebracht wurde und sich während der Reaktion in die Lewis-Säure Eisen (III)-chlorid umwandelt, enthalten. Die obengenannten Prozentangaben beziehen sich jeweils auf das Metallion, nicht auf das Chlorid bzw. sonstige Metallsalz- oder -oxid.

Neben den genannten Zusätzen kann der Katalysator noch weitere Zusätze enthalten, die die Flüchtigkeit insbesondere des Kupfer (II)-chlorids herabsetzen, beispielsweise Alkalimetallchloride wie Kaliumchlorid oder Erdalkalimetallchloride wie Calcium- oder Magnesiumchlorid sowie Zusätze weiterer metallischer Verbindungen, die die Wirksamkeit und/oder Selektivität des Katalysators in bezug auf die Gewinnung von 1,2-Dichlorethan verbessern. Als Beispiele seien genannt Silber, Zink, Chrom, Mangan, seltene Erdmetalle wie Cer, Lanthan, Ytterbium und Neodym ; Platinmetalle wie Rhodium, Platin.

Es können auch Mischungen verschiedener Katalysator- und Katalysatorträger-Teilchen verwendet werden, zum Beispiel mit Kupfersalzen behandeltes Trägermaterial, gemischt mit Teilchen des unbehandelten oder eines anders, beispielsweise mit Eisen (III)-chlorid oder einer anderen Lewis-Säure behandelten Trägermaterials.

Das Verhältnis des Gesamtreaktionsraumes vor Einfüllen des Katalysators zum Schüttvolumen des eingefüllten Katalysators beträgt zweckmäßig etwa 1,1 bis 3, vorzugsweise 1,2 bis 1,7.

Die Strömungsgeschwindigkeit der Gase im Reaktionsraum ist zweckmäßig so hoch, daß mindestens 95 Gew.-%, vorzugsweise 100 Gew.-%, Katalysatorteilchen aufgewirbelt werden. Entsprechend ist die Einspeisung des gegebenenfalls im Kreis geführten Inertgases unter Berücksichtigung der in den Reaktionsraum eingespeisten, an der Reaktion beteiligten Gase zu wählen.

Die mittlere Verweilzeit der in Reaktion befindlichen Gase im Reaktionsraum hängt von der gewählten Reaktionstemperatur ab, wobei im allgemeinen die Verweilzeit umso kürzer sein soll, je höher die Reaktionstemperatur eingestellt wird. Die mittlere Verweilzeit beträgt im allgemeinen 10 bis 100, vorzugsweise 20 bis 70 s, und insbesondere 30 bis 60 s. Sie wird beim kontinuierlichen Betrieb ermittelt aus dem in den Reaktionsraum in einer Sekunde eingespeisten Volumen der Gase bei dem Druck und der Temperatur, die im Reaktionsraum herrschen, in bezug auf das Volumen des gesamten Reaktionsraumes, abzüglich des Eigenvolumens des darin enthaltenen Katalysators und der Einbauten (Kühlrohre ; Temperatur-Meßfühler). Das Eigenvolumen der Katalysatorteilchen wird beispielsweise nach der Flüssigkeitsverdrängungs-Methode (siehe weiter unten) ermittelt.

Bevorzugt wird bei dem erfindungsgemäßen Verfahren soviel Sauerstoff beziehungsweise Sauerstoff enthaltendes Inertgas in den Reaktionsraum eingeführt, daß im Gasgemisch, das den Reaktionsraum verläßt, nach Kondensation der leicht kondensierbaren Reaktionsprodukte (z. B. Wasser und 1,2-Dichlorethan) bei + 10 °C und Abtrennung des Chlorwasserstoffs durch übliche Wäsche noch 2 bis 9, insbesondere 4 bis 7 Vol.-% Sauerstoff enthalten sind. Es ist beispielsweise im Hinblick auf eine Wäsche der Abgase mit organischen, brennbaren Lösungsmitteln zur Abtrennung von Restmengen an 1,2-Dichlorethan von Vorteil, wenn der $O_2$-Gehalt des wie soeben beschrieben vorbehandelten Abgases nicht zu hoch, beispielsweise unter 9 Vol.-%, liegt. Ist eine solche Nachreinigung nicht vorgesehen, kann der Sauerstoffgehalt auch noch höher, beispielsweise bei 10 bis 13 Vol.-% liegen, wobei immer noch sehr gute Ausbeuten an 1,2-Dichlorethan erzielbar sind.

Die Auftrennung und Reinigung des den Reaktionsraum verlassenden Gasgemisches erfolgt, wie weiter oben bereits beschrieben, nach bekannten Verfahren.

Wie gleichfalls oben bereits angedeutet, ermöglicht es das erfindungsgemäße Verfahren einerseits in einer einzigen Reaktionseinheit Ethylen mit besonders guter Ausbeute durch Oxychlorierung der Hauptmenge und Nachchlorierung der Restmenge des Ethylens zu 1,2-Dichlorethan von guter Qualität umzusetzen. Andererseits ermöglicht das neue Verfahren, durch Verwendung nur einer Reaktionseinheit die Gesamtmenge 1,2-Dichlorethan in guter Qualität und Ausbeute für die weitere thermische Zersetzung zur Herstellung von Vinylchlorid bereitzustellen, unter weitgehend vollständiger Wiederverwendung des bei der thermischen Zersetzung anfallenden Chlorwasserstoffs. In der Reaktionseinheit entsteht eine beträchtliche Wärmemenge auf einem Temperaturniveau, das eine günstige Weiterverwendung dieser Wärme, beispielsweise als Mitteldruckdampf, ermöglicht. Das Verfahren benötigt keine komplizierten, kostenintensiven oder störanfälligen apparativen Einrichtungen, es kann in Apparaten durchgeführt werden, die leicht zu reinigen und instandzuhalten sind.

Die Aufteilung der Ethylen-Einleitung in den Reaktionsraum ermöglicht eine gleichmäßigere Temperaturführung und damit verbesserte Beherrschung des Reaktionsablaufes.

Nachfolgende Beispiele sollen die Erfindung näher erläutern :

## Beispiele 1 bis 7

Es wird folgende Apparatur verwendet : Zur Ausführung der Umsetzung von Ethylen zu 1,2-Dichlorethan dient ein senkrecht stehendes Glasrohr mit 80 mm innerem Durchmesser, das unten und oben zu je einer Gaseinström- bzw. Gasausströmöffnung verjüngt ist. Dieses senkrecht stehende Reaktionsrohr enthält unmittelbar über der unteren Einströmöffnung eine Glasfritte, die über den gesamten inneren Querschnitt des Reaktionsrohrs verläuft. In kurzem Abstand über dieser ersten Fritte ist eine zweite Fritte angebracht, deren Fläche etwa den halben Querschnitt des Reaktionsrohrs ausmacht und die in ihrem unteren Teil mit einem Glasrohr verbunden ist, das seitlich durch den Mantel des Reaktionsrohres geführt ist.

Zur Temperierung enthält das Reaktionsrohr eine Glasrohrschlange, deren Anschlüsse ebenfalls seitlich durch den Mantel des Reaktionsrohres geführt sind und die kurz oberhalb der zweiten Fritte beginnt und im Reaktionsrohr soweit nach oben reicht, daß etwa 1/10 der gesamten Länge des Reaktionsrohres im oberen Teil freibleiben. Zwischen der zweiten Fritte und dem oberen Ende des Reaktionsrohres sind über den Mantel des Rohres gleichmäßig verteilt 4 Stutzen angebracht, durch die Temperatur-Meßfühler in das Innere des Reaktionsrohres reichen. In bestimmten Abständen oberhalb der Fritte 2 enthält der Mantel des Reaktionsrohres drei weitere Stutzen, durch die Gaseinleitungsrohre geführt werden können, die bis in die Mitte des Reaktionsrohres reichen, dort senkrecht nach unten gebogen sind und in einer gelochten Kugel enden. Sofern ein Gaseinleitungsrohr durch den Stutzen (A) geführt ist, der in der größten Entfernung von der zweiten Fritte angebracht ist, beträgt der Abstand zwischen der gelochten Kugel und der zweiten Fritte 69 % der gesamten inneren Länge des Reaktionsraumes in dem Reaktionsrohr. Der Reaktionsraum wird gemessen von der Oberfläche der ersten Fritte bis zur Verjüngungsstelle im obersten Teil des Reaktionsrohres. Sofern ein Gaseinleitungsrohr durch den Stutzen (C) geführt ist, der am nächsten an der zweiten Fritte liegt, beträgt der Abstand von der gelochten Kugel des Gaseinleitungsrohrs bis zur zweiten Fritte 17 % der gesamten Länge des Reaktionsraumes im Reaktionsrohr. Wenn ein Gaseinleitungsrohr durch den Stutzen (B) geführt ist, der zwischen den beiden zuletzt beschriebenen Stutzen liegt, beträgt der Abstand von der gelochten Kugel des Gaseinleitungsrohres bis zur zweiten Fritte 53 % der gesamten Länge des Reaktionsraumes im Reaktionsrohr. Der gesamte Mantel des Reaktionsrohres ist mit einer Wärmeisolierschicht versehen.

Oberhalb des Reaktionsrohres ist eine Glaskugel angebracht zwecks Abscheidung von Katalysatorteilchen, die vom Gasstrom mitgerissen werden. Diese Glaskugel wiederum ist über eine absteigende Leitung mit einem Wasserkühler verbunden, an dessen unterem Ende ein Kondensatsammelgefäß mit Ablaßhahn angebracht ist. Das Kondensatsammelgefäß enthält in seinem Oberteil ein Gasabführungsrohr, das wiederum in einen aufsteigenden Solekühler mündet. Die hier kondensierten Bestandteile des Gases fließen in ein zweites Kondensatsammelgefäß mit Ablaßhahn. Die den oberen Teil des Solekühlers verlassenden nichtkondensierbaren Abgase werden durch Waschflaschen geleitet, zur Abscheidung des darin enthaltenen Chlorwasserstoffs. Von dem gewaschenen Abgas werden Proben für eine gaschromatographische Analyse entnommen. Die Kondensate, die sich in den beiden unterhalb der Kühler angebrachten Gefäßen sammeln, werden vereinigt und ebenfalls gaschromatographisch analysiert.

Die Glaskugel, in der die mitgerissenen Katalysatorteilchen abgeschieden werden, sowie das von ihr ausgehende Überleitungsrohr zum Wasserkühler sind mit elektrischen Heizmanschetten versehen. Diese Apparateteile werden während des Betriebes des Reaktors soweit geheizt, daß hier keine Kondensatbildung auftritt.

Das Volumen des Reaktionsraumes im Reaktionsrohr abzüglich der darin enthaltenen Einbauten (Temperierschlange, zweite Fritte, Gaseinleitungskugel sowie Temperaturmeßfühler) beträgt 4 700 cm³.

Zur Durchführung des ersten Beispiels wird das Reaktionsrohr mit 2,8 dm³ (Schüttvolumen) eines Katalysators gefüllt, der aus einem Aluminiumoxidträger besteht und 3,7 Gew.-%, bezogen auf den Katalysator, Kupfer in Form eines Salzes und Spuren Eisen enthält. Der Katalysator hat folgende Siebanalyse :

Teilchen < 20 μ                     25 Gew.-%
Teilchen > 20 μ, jedoch < 70 μ 65 Gew.-%
Teilchen > 70 μ                    10 Gew.-%.

Das Eigenvolumen des Katalysators wird nach der Wasserverdrängungsmethode bestimmt : 1 Meßzylinder mit 2 dm³ Inhalt wird zunächst mit 1 dm³ Katalysatorteilchen gefüllt und dazu 1 dm³ Wasser von 20 °C gegeben. Die Mischung wird etwas geschüttelt und einige Zeit stehengelassen bis keine Gasblasen mehr aufsteigen. Das Volumen der Mischung beträgt nun 1 300 cm³. 1 dm³ (Schüttvolumen) des Katalysators besitzt demnach ein Eigenvolumen der Katalysatorteilchen von 300 cm³. Die gesamte Katalysatorfüllung von 2,8 dm³ hat ein Eigenvolumen von 840 cm³. Der freie Gasraum im Reaktionsrohr beträgt nach Einfüllung des Katalysators noch 3,86 dm³.

Nun wird über das untere Gaseinleitungsrohr durch die erste Fritte Luft in einer Menge von 60 Normal-dm³ pro Stunde geblasen und die Temperierschlange im Reaktionsrohr mit einer Heizflüssigkeit geheizt. Nach etwa 25 Minuten wird im Reaktionsrohr eine Lufttemperatur von 185 °C gemessen, die sich innerhalb der nächsten 5 Minuten nicht mehr ändert. Nun wird die eingeblasene Luftmenge auf 90 Ndm³ · h⁻¹ erhöht und gleichzeitig über die zweite Fritte ein Gemisch von Ethylen in der weiter unten angegebenen Menge und 44 Ndm³ · h⁻¹ Chlorwasserstoffgas eingeleitet. Unmittelbar danach wird auch mit der Einleitung von 22 Ndm³ · h⁻¹ Chlorgas über das mit der Kugel versehene Gaseinleitungsrohr, das in dem von der zweiten Fritte am weitesten entfernten Stutzen (A) angebracht ist, begonnen, gleichzeitig wird Ethylen in der weiter unten angegebenen Menge durch das mit Kugel versehene Gaseinleitungsrohr, das in dem mittleren Stutzen (B) angebracht ist, in das Reaktionsrohr eingespeist. (Der Stutzen, welcher der zweiten Fritte am nächsten liegt, wird nicht benützt, er ist mit einem Stopfen verschlossen). Alle dem Reaktionsrohr zugeführten Gase sind auf 60 °C vorgeheizt.

Bei den einzelnen Beispielen werden insgesamt jeweils 44,5 Ndm³ · h⁻¹ Ethylen in folgenden Teilmengen durch die beiden Einleitungsstellen (zweite Fritte und Stutzen B) dem Reaktionsrohr zugeführt :

| Bei- spiel Nr. | Ethylen über: | | % von der gesamten Ethylenmenge | |
|---|---|---|---|---|
| | zweite Fritte Ndm³h⁻¹ | Stutzen B Ndm³h⁻¹ | erste Teilmenge über zweite Fritte | zweite Teilmenge (Rest) über Stutzen B |
| 1 | 43,4 | 1,1 | 97,5 | 2,5 |
| 2 | 38,9 | 5,6 | 87,4 | 12,6 |
| 3 | 33,4 | 11,1 | 75,0 | 25,0 |
| 4 | 27,8 | 16,7 | 62,5 | 37,5 |
| 5 | 24,5 | 20,0 | 55,0 | 45,0 |
| 6 | 22,3 | 22,2 | 50,1 | 49,9 |
| 7 | 20,0 | 24,5 | 45,0 | 55,0 |

Zusammen mit der Einleitung der Reaktionsgase wird der Wasserkühler mit Wasser von + 14 °C und der Solekühler mit Kühlsole von − 15 °C beschickt. Die Abgaswaschflaschen enthalten Wasser als Waschflüssigkeit.

Nach kurzer Zeit ist die Temperatur im Reaktionsrohr auf 223 °C gestiegen. Sie wird im weiteren Verlauf des Versuches durch Beschickung der Temperierschlange mit Kühlflüssigkeit auf dieser Temperatur gehalten. Das den Solekühler verlassende Abgas hat eine Temperatur von + 10 °C.

Der Versuch wird während 3 Stunden fortgeführt und nach 1/3 und 2/3 dieser Zeit je einmal die Abgaszusammensetzung des gewaschenen Abgases gaschromatographisch ermittelt. Für die Gase Sauerstoff, Kohlenoxid, Kohlendioxid und Ethylen wird ein Wärmeleitfähigkeitsdetektor, für alle anderen nachstehend angegebenen Gase ein Flammionisationsdetektor verwendet. Die Mittelwerte aus beiden Analysen sind für die Beispiele 1 bis 7 in der weiter unten stehenden Tabelle II zusammengefaßt, wobei vom Sauerstoffwert der über die verwendete Luft mitgebrachte Edelgasanteil bereits abgerechnet ist.

Nach Ablauf der Versuchsdauer wird die Gaszufuhr zum Reaktionsrohr beendet und der Katalysator mit Luft (etwa Zimmertemperatur) kaltgeblasen. Das vom Wasser und Solekühler gebildete Kondensat wird vereinigt, gewogen und ebenfalls gaschromatographisch mit einem Flammionisationsdetektor analysiert. Die für die Beispiele 1 bis 7 ermittelten Werte sind in der weiter unten stehenden Tabelle I aufgeführt.

Für die Beispiele 1 bis 7 werden folgende Werte gefunden:

Molverhältnis $HCl : C_2H_4 : Cl_2 : O_2 = 2 : 2,05 : 1 : 0,86$.

Mittlere Verweilzeit der Gase im Reaktionsraum: 40 s. Raum-Zeit-Ausbeute in g Roh-1,2-Dichlorethan $\cdot$ h⁻¹ $\cdot$ dm⁻³, bezogen auf 3,86 dm³ Reaktionsraum.

| Beispiel-Nr. | 1 | 2 | 3 | 4 | 5 | 6 | 7 |
|---|---|---|---|---|---|---|---|
| Raum-Zeit-Ausbeute | 48,7 | 48,4 | 48,3 | 48,0 | 47,8 | 47,6 | 47,4 |

Tabelle I

Gaschromatographische Analyse des kondensierten Roh-1,2-Dichlorethans

| Komponenten | Beispiel Nr. 1 Gew.-% | Beispiel Nr. 2 Gew.-% | Beispiel Nr. 3 Gew.-% | Beispiel Nr. 4 Gew.-% | Beispiel Nr. 5 Gew.-% | Beispiel Nr. 6 Gew.-% | Beispiel Nr. 7 Gew.-% |
|---|---|---|---|---|---|---|---|
| 1,2-Dichlorethan | 98,211 | 98,122 | 98,026 | 97,835 | 97,748 | 97,592 | 97,394 |
| Summe $C_2H_2$ ; $C_2H_4$ ; $C_2H_6$ | 0,001 | 0,001 | 0,001 | 0,001 | 0,001 | 0,001 | 0,002 |
| Vinylchlorid | 0,001 | 0,002 | 0,001 | 0,001 | 0,002 | 0,001 | 0,001 |
| $C_2H_5Cl$ | 0,025 | 0,024 | 0,030 | 0,030 | 0,024 | 0,025 | 0,028 |
| 1,2-Dichlorethylen (trans) | 0,012 | 0,013 | 0,015 | 0,013 | 0,014 | 0,012 | 0,013 |
| 1,1-Dichlorethan | 0,008 | 0,009 | 0,008 | 0,008 | 0,009 | 0,008 | 0,009 |
| $CCl_4$ | 0,047 | 0,030 | 0,042 | 0,032 | 0,047 | 0,029 | 0,035 |
| 1,2-Dichlorethylen (cis) | 0,075 | 0,078 | 0,074 | 0,083 | 0,084 | 0,085 | 0,089 |
| $CHCl_3$ | 0,020 | 0,022 | 0,023 | 0,025 | 0,022 | 0,023 | 0,024 |
| 1,1,2-Trichlorethylen | 0,004 | 0,005 | 0,004 | 0,007 | 0,006 | 0,007 | 0,006 |
| 1,1,2-Trichlorethan | 0,951 | 1,042 | 1,105 | 1,298 | 1,293 | 1,448 | 1,612 |
| 2-Chlorethanol | 0,005 | 0,006 | 0,004 | 0,003 | 0,006 | 0,005 | 0,003 |
| 1,1,2,2-Tetrachlorethan | 0,412 | 0,408 | 0,422 | 0,434 | 0,485 | 0,499 | 0,512 |
| Chloral | 0,225 | 0,234 | 0,240 | 0,225 | 0,255 | 0,258 | 0,265 |

**0 058 976**

Tabelle II

Gaschromatographische Analyse des Abgases nach der Chlorwasserstoff-Wäsche

| Komponenten | Beispiel Nr. 1 Vol.-% | Beispiel Nr. 2 Vol.-% | Beispiel Nr. 3 Vol.-% | Beispiel Nr. 4 Vol.-% | Beispiel Nr. 5 Vol.-% | Beispiel Nr. 6 Vol.-% | Beispiel Nr. 7 Vol.-% |
|---|---|---|---|---|---|---|---|
| $O_2$ | 5,30 | 5,35 | 5,45 | 5,65 | 5,85 | 6,10 | 6,30 |
| CO | 1,65 | 1,70 | 1,70 | 1,65 | 1,60 | 1,60 | 1,58 |
| $CO_2$ | 1,95 | 1,95 | 2,00 | 1,95 | 1,90 | 1,85 | 1,80 |
| $C_2H_4$ | 0,95 | 1,00 | 0,98 | 1,05 | 1,10 | 1,25 | 1,50 |
| Vinylchlorid | 0,001 | 0,002 | 0,001 | 0,001 | 0,001 | 0,001 | 0,002 |
| $C_2H_5Cl$ | 0,011 | 0,012 | 0,012 | 0,013 | 0,014 | 0,015 | 0,016 |
| Leichtsieder | 0,010 | 0,008 | 0,009 | 0,011 | 0,010 | 0,012 | 0,013 |
| 1,2-Dichlorethan | 2,20 | 2,35 | 2,40 | 2,25 | 2,45 | 2,20 | 2,30 |
| Hochsieder | 0,001 | 0,001 | 0,001 | 0,001 | 0,001 | 0,001 | 0,002 |
| 1,1,2-Trichlorethan | 0,009 | 0,008 | 0,009 | 0,012 | 0,013 | 0,013 | 0,015 |
| $Cl_2$ im Abgas | frei | frei | frei | frei | frei | frei | frei |
| $Cl_2$ im Wasser | frei | frei | frei | frei | frei | frei | frei |
| $Cl_2$ im Roh-1,2-Dichlorethan | frei | frei | frei | frei | frei | frei | frei |
| Umsatz, bezogen auf : (in %) | | | | | | | |
| HCl | 97 | 96 | 96 | 95 | 93 | 91 | 90 |
| $C_2H_4$ | 96 | 96 | 95 | 94 | 94 | 93 | 93 |
| $Cl_2$ | 100 | 100 | 100 | 100 | 100 | 100 | 100 |

Beispiele 8 bis 11

Es wird die gleiche Apparatur und nach Art und Menge der gleiche Katalysator verwendet wie in Beispielen 1 bis 7, jedoch mit dem Unterschied, daß ein Gaseinleitungsrohr mit kugelförmigem Ende in dem Stutzen (C) am Reaktionsrohr angeordnet ist, der am nächsten an der zweiten Fritte liegt, so daß, wie oben bereits erwähnt, die Kugel des Gaseinleitungsrohres von der zweiten Fritte in einer Entfernung von 17 % der gesamten Länge des Reaktionsraumes liegt. Der am weitesten entfernt von der zweiten Fritte gelegene Stutzen und der in 53 % der Höhe des Reaktionsraumes von der zweiten Fritte entfernt gelegene Stutzen werden nicht benützt. Sie sind mit Stopfen verschlossen. Durch die untere Öffnung des Reaktionsrohres wird ein Gemisch von 90 $Ndm^3 \cdot h^{-1}$ Luft und 22 $Ndm^3 \cdot h^{-1}$ Chlor eingeleitet, das über die erste Fritte in den Reaktionsraum eintritt. Über die zweite Fritte wird ein Teil des Ethylens und über das Rohr mit dem kugelförmigen Ende 44 $Ndm^3 \cdot h^{-1}$ Chlorwasserstoffgas und der Rest des Ethylens eingeleitet. Bei den einzelnen Beispielen werden insgesamt jeweils 45 $Ndm^3 \cdot h^{-1}$ Ethylen in folgenden Teilmengen durch die beiden Einleitungsstellen (zweite Fritte und Stutzen C) dem Reaktionsrohr zugeführt :

| Bei-spiel Nr. | Ethylen über | | % von der gesamten Ethylenmenge | |
|---|---|---|---|---|
| | zweite Fritte $Ndm^3h^{-1}$ | Stutzen C $Ndm^3h^{-1}$ | erste Teilmenge über zweite Fritte | zweite Teilmenge (Rest) über Stutzen C |
| 8 | 43,9 | 1,1 | 97,6 | 2,4 |
| 9 | 33,7 | 11,3 | 74,9 | 25,1 |
| 10 | 22,5 | 22,5 | 50,0 | 50,0 |
| 11 | 20,3 | 24,7 | 45,1 | 54,9 |

Die Temperatur im Reaktionsraum wird auf 222 °C konstant gehalten, die Versuchsdauer beträgt 3 Stunden. Im übrigen wird weiterverfahren, wie in Beispiel 1 beschrieben.

Für die Beispiele 8 bis 11 werden folgende Werte gefunden :

Molverhältnis : $C_2H_4$ : HCl : $Cl_2$ : $O_2$ = 2 : 1,95 : 0,98 : 0,84.

Mittlere Verweilzeit der Gase im Reaktionsraum : 40 s. Raum-Zeit-Ausbeute in g Roh-1,2-Dichlorethan $\cdot h^{-1} \cdot dm^{-3}$, bezogen auf 3,86 $dm^3$ Reaktionsraum :

| Beispiel-Nr. | 8 | 9 | 10 | 11 |
|---|---|---|---|---|
| Raum-Zeit-Ausbeute | 48,7 | 48,5 | 48,4 | 48,6 |

9

# 0 058 976

Roh-1,2-Dichlorethan- und Abgas-Analysen siehe untenstehende Tabellen III und IV.

Bei allen beschriebenen Beispielen (Nr. 1 bis 11) wird bei normalen Atmosphärendruck von 97,3 kPa gearbeitet.

### Tabelle III

### Gaschromatographische Analyse des kondensierten Roh-1,2-Dichlorethans

| Komponenten | Beispiel Nr. 8 Gew.-% | Beispiel Nr. 9 Gew.-% | Beispiel Nr. 10 Gew.-% | Beispiel Nr. 11 Gew.-% |
|---|---|---|---|---|
| 1,2-Dichlorethan | 98,327 | 98,264 | 98,258 | 98,207 |
| Summe $C_2H_2$ ; $C_2H_4$ ; $C_2H_6$ | 0,002 | 0,001 | 0,001 | 0,002 |
| Vinylchlorid | 0,003 | 0,003 | 0,002 | 0,003 |
| $C_2H_5Cl$ | 0,011 | 0,020 | 0,020 | 0,022 |
| 1,2-Dichlorethylen (trans.) | 0,018 | 0,020 | 0,018 | 0,017 |
| 1,1-Dichlorethan | 0,009 | 0,009 | 0,009 | 0,010 |
| $CCl_4$ | 0,082 | 0,089 | 0,094 | 0,088 |
| 1,2-Dichlorethylen (cis) | 0,084 | 0,088 | 0,095 | 0,099 |
| $CHCl_3$ | 0,054 | 0,055 | 0,056 | 0,063 |
| 1,1,2-Trichlorethylen | 0,005 | 0,005 | 0,006 | 0,006 |
| 1,1,2-Trichlorethan | 0,709 | 0,729 | 0,749 | 0,766 |
| 2-Chlorethanol | 0,003 | 0,003 | 0,003 | 0,003 |
| 1,1,2,2-Tetrachlorethan | 0,340 | 0,355 | 0,351 | 0,369 |
| Chloral | 0,350 | 0,354 | 0,335 | 0,340 |

### Tabelle IV

### Gaschromatographische Analyse des Abgases nach der Chlorwasserstoff-Wäsche

| Komponenten | Beispiel Nr. 8 Vol.-% | Beispiel Nr. 9 Vol.-% | Beispiel Nr. 10 Vol.-% | Beispiel Nr. 11 Vol.-% |
|---|---|---|---|---|
| $O_2$ | 1,50 | 1,50 | 1,40 | 1,50 |
| CO | 2,50 | 2,60 | 2,60 | 2,55 |
| $CO_2$ | 3,20 | 3,30 | 3,35 | 3,30 |
| $C_2H_4$ | 0,54 | 0,48 | 0,44 | 0,48 |
| Vinylchlorid | 0,002 | 0,002 | 0,001 | 0,002 |
| $C_2H_5Cl$ | 0,012 | 0,013 | 0,014 | 0,011 |
| Leichtsieder | 0,008 | 0,007 | 0,009 | 0,008 |
| 1,2-Dichlorethan | 2,40 | 2,35 | 2,45 | 2,40 |
| Hochsieder | < 0,001 | < 0,001 | < 0,001 | < 0,001 |
| 1,1,2-Trichlorethan | 0,004 | 0,003 | 0,005 | 0,004 |
| $Cl_2$ im Abgas | frei | frei | frei | frei |
| $Cl_2$ im Wasser | frei | frei | frei | frei |
| $Cl_2$ im Roh-1,2-Dichlorethan | frei | frei | frei | frei |
| Umsatz, bezogen auf (in %) : | | | | |
| HCl | 97 | 97 | 97 | 97 |
| $C_2H_4$ | 95 | 95 | 95 | 95 |
| $Cl_2$ | 100 | 100 | 100 | 100 |

### Beispiele 12 und 13

Die hierfür verwendete Apparatur ist analog der bei den Beispielen 1 bis 7 verwendeten aufgebaut, jedoch mit dem Unterschied, daß als Reaktionsraum ein senkrecht stehendes Nickelrohr mit 50 mm innerem Durchmesser verwendet wird, das ähnlich dem Glasrohr der für Beispiele 1 bis 7 verwendeten Apparatur ausgestattet ist, mit folgenden Unterschieden : Es sind nur drei Temperatur-Meßstellen über den Mantel des Reaktionsrohres gleichmäßig verteilt vorhanden. Von unten nach oben gesehen sind die ersten beiden Fritten angebracht, wie in der Apparatur für Beispiele 1 bis 7 beschrieben. Dann folgt eine dritte Fritte und darüber eine vierte. Der Abstand von der zweiten Fritte beträgt bei der dritten Fritte 41 %, bei der vierten Fritte 47 % der gesamten inneren Länge des Reaktionsraumes. Auch am Kopf des Rohres,

10

unmittelbar vor der Verjüngung, ist eine fünfte Fritte eingebaut, die zur Druckminderung und zum Zurückhalten mitgerissener Katalysatorteilchen verwendet wird, die in der Glasapparatur hierfür vorgesehene Kugel entfällt. Am Reaktorausgang ist ein Druckminderungsventil angebracht. Das Gaseinleitungsrohr mit kugelförmigem Kopf ist in den Reaktor fest eingebaut, wobei der Abstand von der Kugel bis zur zweiten Fritte (von unten) 56 % der Länge des gesamten Reaktionsraumes, gemessen zwischen der untersten und der obersten Fritte im Rohr, beträgt. Im oberen Teil des Rohres ist eine Druckmeßvorrichtung angebracht.

Der Inhalt des Reaktionsraumes abzüglich dem Volumen der darin befindlichen Einbauten (Temperierschlange, Gaseinleitungsrohre mit Fritte bzw. Kugelkopf, Temperaturmeßfühler) beträgt 1 500 cm³. Am Reaktorausgang nach dem Druckminderventil sind Wasser- und Solekühler sowie Chlorwasserstoff-Wäscher angeschlossen, wie bei den Beispielen 1 bis 7 beschrieben.

Das Nickelrohr wird mit 1 dm³ (Schüttvolumen) eines Katalysators gefüllt, der Kupferchlorid auf Aluminiumoxid enthält und einen Kupfergehalt von 4,5 Gew.-%, bezogen auf den Katalysator, aufweist. Der Katalysator enthält kein Eisen und hat folgende Siebanalyse :

Teilchen < 20 μ                    22 Gew.-%
Teilchen > 20 μ jedoch < 70 μ    67 Gew.-%
Teilchen > 70 μ                    11 Gew.-%

Das Eigenvolumen des Katalysators wird, wie oben beschrieben, nach der Wasserverdrängungsmethode zu 310 cm³ ermittelt. Der für die Gasreaktion zur Verfügung stehende freie Raum beträgt 1 190 cm³.

Durch die unterste Gaszuführungsleitung werden zunächst 60 $Ndm^3 \cdot h^{-1}$ Luft durch die erste (unterste) Fritte unter Druck geblasen und der Reaktor mittels der Temperierschlange auf 240 °C aufgeheizt. Durch Regulierung des Druckminderventils am Kopf des Reaktors wird im Reaktor ein Druck von 500 kPa eingestellt. Nach einer halben Stunde ist Temperatur und Druckkonstanz im Reaktionsrohr erreicht. Nun wird die eingeblasene Luftmenge auf 90 $Ndm^3 \cdot h^{-1}$ erhöht und durch die zweite, dritte und vierte Fritte folgende Gasmengen eingeblasen :

| | über 2. Fritte | | über 3. Fritte | über 4. Fritte | % von der gesamten Ethylenmenge | |
| Beisp. Nr. | $Ndm^3h^{-1}$ | | $Ndm^3h^{-1}C_2H_4$ | $Ndm^3h^{-1}Cl_2$ | erste Teilmenge über 2. Fritte | zweite Teilmenge über 3. Fritte |
| | $C_2H_4$ + HCl | | | | | |
|---|---|---|---|---|---|---|
| 12 | 26 | 50 | 25 | 25 | 51 | 49 |
| 13 | 38 | 50 | 13 | 25 | 74,5 | 25,5 |

Die Temperatur im Reaktionsraum steigt an und wird durch Einführung eines Kühlmediums in die Temperierschlange bei Beispiel 12 auf 280 °C, bei Beispiel 13 auf 265 °C eingestellt und während weiterer 3 Stunden konstant gehalten.

Die in den Reaktionsraum eingeführten Gase werden auf 60 °C vorgeheizt. Nach Ablauf der 3 Stunden wird die Gaszufuhr unterbrochen und der Katalysator im Reaktionsrohr mit Luft (etwa Zimmertemperatur) kaltgeblasen.

Während der gesamten Versuchsdauer ist der Wasserkühler mit Wasser von + 12 °C und der Solekühler mit Kühlsole von − 20 °C durchflossen. Das Abgas, das den Solekühler verläßt, hat eine Temperatur von + 11 °C.

Während und nach dem jeweiligen Versuch erfolgt die Probenahme und Probe-Auswertung wie unter Beispiel 1 bis 7 beschrieben. Die ermittelten Werte sind in nachfolgenden Tabellen V und VI aufgeführt :
Für die Beispiele 12 und 13 werden folgende Werte gefunden :
Molverhältnis : HCl : $C_2H_4$ (gesamt) : $Cl_2$ : $O_2$ = 2 : 2,04 : 1 : 0,75
Mittlere Verweilzeit der Gase im Reaktionsraum :
Beispiel 12 : 55 s ; Beispiel 13 : 58 s.
Raum-Zeit-Ausbeute in g Roh-1,2-Dichlorethan : $\cdot h^{-1} \cdot dm^{-3}$, bezogen auf 1,19 dm³ Reaktionsraum.
Beispiel 12 : 181 ; Beispiel 13 : 182.

### Tabelle V

Gaschromatographische Analyse des kondensierten Roh-1,2-Dichlorethans

| Komponenten | Beispiel Nr. 12 Gew.-% | Beispiel Nr. 13 Gew.-% |
|---|---|---|
| 1,2-Dichlorethan | 97,083 | 97,779 |
| Summe $C_2H_2$ ; $C_4H_4$ ; $C_2H_6$ | 0,001 | 0,001 |
| Vinylchlorid | 0,028 | 0,022 |
| $C_2H_5Cl$ | 0,068 | 0,064 |
| 1,2-Dichlorethylen (trans) | 0,016 | 0,011 |
| 1,1-Dichlorethan | 0,006 | 0,006 |
| $CCl_4$ | 0,048 | 0,054 |
| 1,2-Dichlorethylen (cis) | 0,042 | 0,038 |
| $CHCl_3$ | 0,015 | 0,017 |
| 1,1,2-Trichlorethylen | 0,003 | 0,005 |
| 1,1,2-Trichlorethan | 1,567 | 1,012 |
| 2-Chlorethanol | 0,038 | 0,042 |
| 1,1,2,2-Tetrachlorethan | 0,756 | 0,643 |
| Chloral | 0,310 | 0,290 |

### Tabelle VI

Gaschromatographische Analyse des Abgases nach der Chlorwasserstoff-Wäsche

| Komponenten | Beispiel Nr. 12 Vol.-% | Beispiel Nr. 13 Vol.-% |
|---|---|---|
| $O_2$ | 1,0 | 2,4 |
| CO | 2,7 | 2,1 |
| $CO_2$ | 3,1 | 2,8 |
| $C_2H_4$ | 0,12 | 0,18 |
| Vinylchlorid | 0,040 | 0,025 |
| $C_2H_5Cl$ | 0,056 | 0,048 |
| Leichtsieder | 0,035 | 0,036 |
| 1,2-Dichlorethan | 2,65 | 2,25 |
| Hochsieder | 0,01 | 0,005 |
| 1,1,2-Trichlorethan | 0,005 | 0,004 |
| $Cl_2$ im Abgas | frei | frei |
| $Cl_2$ im Wasser | frei | frei |
| $Cl_2$ im Roh-1,2-Dichlorethan | frei | frei |
| Umsatz bezogen auf (in %) : | | |
| HCl | 99 | 99 |
| $Cl_2$ | 100 | 100 |
| $C_2H_4$ | 95,3 | 96,4 |

**Ansprüche**

1. Verfahren zur Herstellung von 1,2-Dichlorethan aus Ethylen durch Umsetzung mit Chlorwasserstoff und Sauerstoff enthaltenden Inertgasen bei 180 bis 300 °C und 0,1 bis 1,1 MPa sowie durch Umsetzung mit Chlor in der Gasphase in Gegenwart eines kupfer- oder kupfer- und eisensalzhaltigen festen Katalysators mit anschließender Abkühlung und destillativer Auftrennung des Reaktionsgemisches, wobei beide Chlorierungsreaktionen in einem gemeinsamen Reaktionsraum, der aufgewirbelte Katalysatorteilchen enthält, nacheinander durchgeführt werden, und die im gesamten Reaktionsraum gebildete Wärme durch indirekte Kühlung mit einem flüssigen und/oder gasförmigen Wärmeübertragungsmedium abgeführt wird, dadurch gekennzeichnet, daß 98 bis 40 % der Gesamtmenge des in den gemeinsamen Reaktionsraum eingeleiteten Ethylen-Volumens in die Zone des Reaktionsraumes eingeleitet wird, in der die erste Chlorierungsreaktion stattfindet, und die restlichen 2 bis 60 % der Gesamtmenge

des in den gemeinsamen Reaktionsraum eingeleiteten Ethylen-Volumens in die Zone des Reaktionsraumes eingeleitet wird, in der die zweite Chlorierungsreaktion stattfindet.

2. Verfahren nach Anspruch 1, dadurch gekennzeichnet, daß 95 bis 60 % der Gesamtmenge des in den gemeinsamen Reaktionsraum eingeleiteten Ethylenvolumens in die Zone des Reaktionsraumes eingeleitet wird, in der die erste Chlorierungsreaktion stattfindet und die restlichen 5 bis 40 % der Gesamtmenge des in den gemeinsamen Reaktionsraum eingeleiteten Ethylenvolumens in die Zone des Reaktionsraumes eingeleitet wird, in der die zweite Chlorierungsreaktion stattfindet.

3. Verfahren nach Anspruch 1, oder 2, dadurch gekennzeichnet, daß der Reaktionsraum kontinuierlich von den umzusetzenden Gasen durchströmt wird, wobei in eine in Strömungsrichtung der Gase zuerst gelegene Zone des Reaktionsraumes getrennt oder teilweise getrennt voneinander eingeleitet werden : Chlorwasserstoff, eine Teilmenge des Ethylens und Sauerstoff enthaltendes Inertgas und in eine in Strömungrichtung nachfolgende Zone des Reaktionsraumes getrennt voneinander die restliche Teilmenge des Ethylens und Chlor in folgenden Mengenverhältnissen : 2 mol HCl, 1,8 bis 2,2 mol Gesamtmenge des $C_2H_4$, 0,5 bis 0,6 mol $O_2$ und 0,79 bis 1,2 mol $Cl_2$, wobei die Chlormenge so bemessen wird, daß im Gasgemisch, das den Reaktionsraum verläßt, weniger als 0,001 Gew.-% freies, elementares Chlor gefunden werden.

4. Verfahren nach Ansprüchen 1 oder 2, dadurch gekennzeichnet, daß der Reaktionsraum kontinuierlich von den umzusetzenden Gasen durchströmt wird, wobei in eine in Strömungsrichtung der Gase zuerst gelegene Zone des Reaktionsraumes eingeleitet werden : Sauerstoff enthaltendes Inertgas und Chlor sowie getrennt davon eine Teilmenge des Ethylens und in eine in Strömungsrichtung nachfolgende Zone des Reaktionsraumes Chlorwasserstoff und die restliche Teilmenge des Ethylens in folgenden Mengenverhältnissen : 2 mol Gesamtmenge des $C_2H_4$, 0,9 bis 1,2 mol $Cl_2$, 1,6 bis 2,3 mol HCl und 0,35 bis 1,3 mol $O_2$, wobei die Sauerstoff- oder Chlorwasserstoff-Menge so bemessen werden, daß im Gasgemisch, das den Reaktionsraum verläßt, weniger als 0,001 Gew.-% freies, elementares Chlor gefunden werden.

## Claims

1. A process for the manufacture of 1,2-dichloroethane from ethylene by reaction with hydrogen chloride and inert gases containing oxygen, at 180 to 300 °C and a pressure of 0.1 to 1.1 MPa, and by reaction with chlorine in the gas phase in the presence of a solid catalyst containing copper salts or copper and iron salts, with subsequent cooling and separation by distillation of the reaction mixture, both chlorination reactions being carried out successively in a common reaction space containing fluidised catalyst particles, and the heat formed in the whole reaction space being removed by indirect cooling with a liquid and/or gaseous heat transfer medium, characterised by introducing, into the zone of the reaction space in which the first chlorination reaction takes place, 98 to 40 % of the total quantity of the volume of ethylene introduced into the common reaction space, and introducing, into the zone of the reaction space, in which the second chlorination reaction takes place, the remaining 2 to 60 % of the total quantity of the volume of ethylene introduced into the common reaction space.

2. The process as claimed in claim 1, characterised in that 95 to 60 % of the total quantity of the volume of ethylene introduced into the common reaction space is introduced into the zone of the reaction space in which the first chlorination reaction takes place, and the remaining 5 to 40 % of the total quantity of the volume of ethylene introduced into the common reaction space is introduced into the zone of the reaction space in which the second chlorination takes place.

3. The process as claimed in claims 1 or 2, characterised in that the gases to be reacted flow continuously through the reaction space, hydrogen chloride, a fraction of the ethylene, and inert gas containing oxygen being introduced, separately, or in part separately, from one another into a initially-situated zone of the reaction space, in the direction of flow of the gases, and the residual fraction of the ethylene, and chlorine being introduced, separately from one another, into a subsequent zone of the reaction space, in the direction of flow, in the following ratios : 2 moles of HCl, 1.8 to 2.2. moles, total quantity, of $C_2H_4$, 0.5 to 0.6 mole of $O_2$ and 0.79 to 1.2 moles of $Cl_2$, the quantity of chlorine being such that less than 0.001 % by weight of free, elementary chlorine is found in the gas mixture leaving the reaction space.

4. The process as claimed in claims 1 or 2, characterised in that the gases to be reacted flow continuously through the reaction space, inert gas containing oxygen, and chlorine, and also, separately therefrom, a fraction of the ethylene being introduced into an initially-situated zone of the reaction space, in the direction of flow of the gases, and hydrogen chloride and the remaining fraction of the ethylene being introduced into a subsequent zone of the reaction space, in the direction of flow, in the following ratios : 2 moles, total quantity, of $C_2H_4$, 0.9 to 1.2 moles of $Cl_2$, 1.6 to 2.3 moles of HCl and 0.35 to 1.3 moles of $O_2$, the quantity of oxygen or hydrogen chloride being such that less than 0.001 % by weight of free, elementary chlorine is found in the gas mixture leaving the reaction space.

## Revendications

1. Procédé de préparation du dichloro-1,2 éthane à partir de l'éthylène par réaction avec le chlorure d'hydrogène et des gaz inertes contenant de l'oxygène, à 180 jusqu'à 300 °C et de 0,1 jusqu'à 1,1 MPa, ainsi que par réaction du chlore en phase gazeuse, en présence d'un catalyseur solide contenant du sel de cuivre ou du sel de cuivre et de fer, avec refroidissement subséquent et séparation par distillation du mélange réactionnel, selon lequel on effectue successivement les deux réactions de chloration dans un espace réactionnel commun qui contient des particules du catalyseur en suspension fluidisée et l'on évacue, par refroidissement indirect à l'aide d'un milieu liquide et/ou gazeux de transmission de chaleur, la chaleur formée dans l'ensemble de l'espace de réaction, procédé caractérisé en ce qu'on introduit 98 à 40 %, de la quantité totale du volume d'éthylène introduit dans l'ensemble de l'espace de réaction, dans la zone de l'espace de réaction dans laquelle se produit la première réaction de chloration, et l'on introduit les 2 à 60 % restants de la quantité totale du volume d'éthylène introduit dans l'ensemble de l'espace de réaction dans la zone de l'espace de réaction dans laquelle se produit la seconde réaction de chloration.

2. Procédé selon la revendication 1, caractérisé en ce qu'on introduit 95 à 60 % de la quantité totale du volume d'éthylène introduit dans l'ensemble de l'espace de réaction, dans la zone de l'espace de réaction dans laquelle se produit la première réaction de chloration, et l'on introduit les 5 à 40 % restants de la quantité totale du volume d'éthylène introduit dans l'ensemble de l'espace de réaction, dans la zone de l'espace de réaction dans laquelle se produit la seconde réaction de chloration.

3. Procédé selon la revendication 1 ou 2, caractérisé en ce que l'espace de réaction est continuellement parcouru par les gaz à faire réagir, avec introduction séparée, ou partiellement séparée les uns des autres, dans une zone de l'espace de réaction située en premier, dans le sens d'écoulement des gaz : du chlorure d'hydrogène, une quantité partielle de l'éthylène et du gaz inerte contenant de l'oxygène et, dans une zone suivante, dans le sens de l'écoulement, de l'espace de réaction, séparément l'un de l'autre, la quantité partielle résiduelle de l'éthylène et du chlore selon les rapports suivants : 2 moles de HCl, 1,8 à 2,2 moles de quantité totale du $C_2H_4$, 0,5 à 0,6 mole de $O_2$ et 0,79 à 1,2 mole de $Cl_2$, la quantité du chlore étant introduite de façon mesurée de manière que l'on trouve moins de 0,001 % en poids de chlore élémentaire libre dans le mélange gazeux sortant de l'espace de réaction.

4. Procédé selon les revendications 1 ou 2, caractérisé en ce que l'espace de réaction est continuellement parcouru par les gaz destinés à réagir, et l'on introduit dans une zone de l'espace de réaction située en premier dans le sens de l'écoulement des gaz : du gaz inerte contenant de l'oxygène, et du chlore ainsi que, séparément, une quantité partielle de l'éthylène et, dans une zone suivante, dans le sens de l'écoulement, de l'espace de réaction, on introduit du chlorure d'hydrogène et la quantité partielle restante de l'éthylène selon les rapports suivants : 2 moles de quantité totale du $C_2H_4$, 0,9 à 1,2 mole de $Cl_2$, 1,6 à 2,3 moles de HCl et 0,35 à 1,3 mole de $O_2$, l'introduction de l'oxygène ou du chlorure d'hydrogène étant mesurée de façon que l'on trouve moins de 0,001 % en poids de chlore élémentaire libre dans le mélange gazeux sortant de l'espace de réaction.